# EUROPÄISCHE PATENTSCHRIFT

(11) **EP 3 662 080 B1**
(45) Veröffentlichungstag und Bekanntmachung des Hinweises auf die Patenterteilung: **05.01.2022**
(21) Anmeldenummer: 18745629.8
(22) Anmeldetag: 30.07.2018
(51) Int. Cl.: C12Q 1/6806, C12N 15/10, A61B 5/15

(54) **VERFAHREN UND ZUSAMMENSETZUNG ZUR STABILISIERUNG ZELLFREIER NUKLEINSÄUREN UND ZELLEN**
METHOD AND COMPOSITION FOR THE STABILISATION OF CELL-FREE NUCLEIC ACIDS AND CELLS
PROCÉDÉ ET COMPOSITION DE STABILISATION D'ACIDES NUCLÉIQUES EXEMPT DE CELLULES ET DES CELLULES

(30) Priorität: 02.08.2017 EP 17184585
(43) Veröffentlichungstag der Anmeldung: 10.06.2020
(73) Patentinhaber: Sarstedt AG & Co. KG, 51582 Nümbrecht (DE)
(72) Erfinder: KÄMPER, Martin, 51766 Engelskirchen-Osberghausen (DE); KINITZ, Tim, 42659 Solingen (DE)
(74) Vertreter: Taruttis, Stefan Georg
(86) Internationale Anmeldenummer: PCT/EP2018/070633
(87) Internationale Veröffentlichungsnummer: WO 2019/025387

(56) Entgegenhaltungen:
- WO-A1-2007/073397
- WO-A1-2013/045458
- WO-A2-02/056030
- WO-A2-03/018757
- WO-A2-03/095974
- WO-A2-2013/123030
- Bd Vacutainer: "Helping all people live healthy lives", , 1. Januar 2006 (2006-01-01), XP055405080, Gefunden im Internet: URL:https://iti.stanford.edu/content/dam/s m/iti/documents/himc/immunoassays/BDVacuta inerTubeGuide.pdf [gefunden am 2017-09-08]
- Bd Diagnostics ET AL: "Product Catalogue", , 1. Januar 2014 (2014-01-01), XP055405100, Gefunden im Internet: URL:http://www.bd.com/resource.aspx?idx=30 770 [gefunden am 2017-09-08]
- SAMINATHAN M ET AL: "Polyamine structural effects on the induction and stabilization of liquid crystalline DNA: potential applications to DNA packaging, gene therapy and polyamine therapeutics", NUCLEIC ACIDS RESEARCH, OXFORD UNIVERSITY PRESS, GB, vol. 30, no. 17, 1 January 2002 (2002-01-01), pages 3722-3731, XP002700434, ISSN: 0305-1048, DOI: 10.1093/NAR/GKF503
- WONG DAVID ET AL: "Optimizing blood collection, transport and storage conditions for cell free DNA increases access to prenatal testing", CLINICAL BIOCHEMISTRY, ELSEVIER INC, US, CA, vol. 46, no. 12, 30 April 2013 (2013-04-30), pages 1099-1104, XP028679416, ISSN: 0009-9120, DOI: 10.1016/J.CLINBIOCHEM.2013.04.023
- TORO PATRICIA VALDA ET AL: "Comparison of cell stabilizing blood collection tubes for circulating plasma tumor DNA", CLINICAL BIOCHEMISTRY, ELSEVIER INC, US, CA, vol. 48, no. 15, 31 July 2015 (2015-07-31) , pages 993-998, XP029291524, ISSN: 0009-9120, DOI: 10.1016/J.CLINBIOCHEM.2015.07.097

## Beschreibung

Die vorliegende Erfindung betrifft ein Verfahren und die Verwendung einer Zusammensetzung zur Stabilisierung zellfreier Nukleinsäuren, insbesondere von zellfreier DNA und/oder RNA, sowie zusätzlich zur Stabilisierung von Zellen aus biologischen Proben, insbesondere Vollblut oder Plasma oder Urin, sowie die Verwendung der Zusammensetzung als Stabilisierungsmittel für zellfreie Nukleinsäuren und Zellen in einer biologischen Probe. Die Zusammensetzung kann einer zellfreien Probe oder einer zellhaltigen Probe zugemischt werden, beispielsweise durch eine Blutentnahmeröhre, in die unmittelbar Vollblut eingezogen wurde und in welcher die Zusammensetzung vorgelegt ist, z.B. zu 20% des maximalen Volumens an Blut vorgelegt, für das die Blutentnahmeröhre eingerichtet ist. Für Urin als biologische Probe kann die Zusammensetzung in einem Probebehälter vorgelegt sein oder der darin eingefüllten Probe zugemischt werden, wobei jeweils bevorzugt ist, die Zusammensetzung in einem vorbestimmten Volumenverhältnis zur Probe bzw. für ein vorbestimmtes Probenvolumen vorzulegen bzw. zuzumischen.

Das Verfahren und die Verwendung einer Zusammensetzung haben den Vorteil, in der biologischen Probe enthaltene zellfreie Nukleinsäuren zu stabilisieren, insbesondere für die weitere Analyse, beispielsweise mittels Hybridisierung, Sequenzierung oder Amplifizierung, optional mit vorheriger Isolierung, z.B. mittels Adsorption an ein Adsorptionsmittel für Nukleinsäuren und anschließender Elution. Die Stabilisierung zellfreier Nukleinsäuren ist insbesondere die Aufrechterhaltung der Menge und Struktur der zellfreien Nukleinsäuren, was auch als deren Integrität bezeichnet werden kann.

Die Verwendung der Zusammensetzung und das Verfahren haben bevorzugt auch die Wirkung, in der Probe enthaltene Zellen zu stabilisieren, z.B. gegen eine spontane Lyse, so dass zum einen in der entnommenen Probe die in Zellen enthaltenen Nukleinsäuren nicht bzw. in verringertem Maße freigesetzt werden und sich nicht mit zellfreien Nukleinsäuren der Probe mischen, und zum anderen aus der Probe Zellen abgetrennt werden können, um die Zellen im Wesentlichen ohne Beeinträchtigung durch Lyse, und z.B. frei von zellfreien Nukleinsäuren, analysieren zu können. Dabei können Zellen solche des Spenders der Probe sein, also körpereigene Zellen, z.B. Epithelzellen und/oder Tumorzellen, und Zellen können körperfremde Zellen sein, z.B. Bakterien, Pilze bzw. Hefen oder Viren.

Die Analyse von Zellen, die aus einer Mischung der Zusammensetzung mit einer biologischen Probe abgetrennt sind, kann die Analyse von intrazellulären und/oder von oberflächengebundenen Proteinen, z.B. durch immunologische Analysen umfassen. Die Zusammensetzung führt bevorzugt zu einer Stabilisierung oberflächengebundener Proteine auf Zellen.

### Stand der Technik

Die WO 2013/123030 A2 beschreibt zur Stabilisierung von Vollblut für die spätere Analyse zellfreier DNA das Einmischen einer Formaldehyd freisetzenden Verbindung, insbesondere von Diazolidinyl-Harnstoff oder Imidazolidinyl-Harnstoff in Kombination mit einem Fänger zur Entfernung freien Formaldehyds, z.B. von Aminosäuren, insbesondere Glycin, Alkylaminen, Polyaminen, jeweils in Verbindung mit EDTA als Antikoagulans.

Umetani et al., Clinical Chemistry 52:6, 1062-1069 (2006) beschreibt zur Analyse der Stabilität von zellfreier DNA in Serum durch quanitative PCR-Amplifikation zweier Abschnitte aus ALU-Repeatsequenzen, von denen der eine Abschnitt von 115 bp (ALU115) innerhalb des anderen Abschnitts von 247 bp (ALU247) liegt. Das Verhältnis der Mengen der Amplifikate bildet ein Maß für die Qualität der DNA.

Ein Quotient von PCR-Amplifikaten von ALU 247 / ALU 115 von 1, also gleiche Mengen ALU 247 und ALU 115, wird als charakteristisch für gDNA angesehen, da eine solche Probe neben den kleinen Templates auch die längeren Templates enthält. Für zellfreie (cf) DNA geben Hao, T. B., Shi, W., Shen, X. J., Qi, J., Wu, X. H., Wu, Y., Tang, Y. Y., Ju, S. Q. "Circulating cell-free DNA in Serum as a biomarker for diagnosis and prognostic prediction of colorectal cancer", British Journal of Cancer 111, 1482 - 1489 (2014) einen ALU 247 / ALU 115 - Quotienten von ca. 0,3 an.

Die WO 2013/045458 beschreibt zur Stabilisierung von Vollblut eine Mischung aus Dihydroxyaceton als hypertonischen Zusatz, N,N-Dimethylformamid, N,N-Diethylformamid, N,N-Dimethylacetamid oder N,N-Diethylacetatmid als Stabilisierungsmittel für extrazelluläre Nukleinsäuren, bevorzugt einem Chelatbildner als Antikoagulans und einem Inhibitor der Apoptose, der insbesondere ein Caspase-Inhibitor ist.

Die WO 03/018757 A2 betrifft nur generell die Stabilisierung der Zellen in einer biologischen Probe mit einer Zusammensetzung, die ein Anticoagulans, einen Formaldehyddonor, z. B. Methenamin, enthält, sowie optional Polyethylenglycol.

Die WO 2007/073397 A1 beschreibt eine pharmazeutische Zusammensetzung zur Behandlung von Blasenerkrankungen mit einem anionischen Polysaccharid und einem Betäubungsmittel in Puffer, die als antibakterielles Mittel Methenamin (entspricht Urotropin) enthalten kann.

### Aufgabe der Erfindung

Die Aufgabe der Erfindung liegt in der Bereitstellung einer alternativen Verwendung einer Zusammensetzung und eines alternativen Verfahrens zur Stabilisierung von zellfreien Nukleinsäuren, z.B. DNA, oder von Zellen in biologischen Proben, insbesondere im Vollblut oder Urin, für die spätere Analyse, wobei die Zusammensetzung bevorzugt lagerstabil ist und weiter bevorzugt eine geringere Anzahl verschiedener Inhaltsstoffe als bekannte Mittel aufweist.

### Beschreibung der Erfindung

Die Erfindung löst die Aufgabe mit den Merkmalen der Ansprüche und insbesondere mit der Verwendung einer Zusammensetzung als Stabilisierungsmittel und einem Verfahren zur Stabilisierung biologischer Proben, insbesondere Vollblut oder Urin, insbesondere zur Stabilisierung des Gehalts und der Integrität zellfreier Nukleinsäuren und zur Stabilisierung des Gehalts und der Integrität von Zellen. Die Zusammensetzung weist in wässriger Lösung zumindest eine Puffersubstanz auf, die auf einen pH-Wert von 7 oder darunter, bevorzugt 3,5 bis 7,0 puffert, und insbesondere eingerichtet ist, die Mischung aus der Zusammensetzung und der biologischen Probe auf diesen pH-Wert zu puffern, zumindest einen Gerinnungshemmer und/oder zumindest einen Chelatbildner, insbesondere zumindest einen Chelatbildner für zweiwertige Kationen, bevorzugt für Calciumionen, und Urotropin auf, optional PEG, oder besteht daraus, insbesondere zur Verwendung als Stabilisierungsmittel für Vollblut. Da Chelatbildner, insbesondere EDTA, auch Gerinnungshemmer sind, können für die Zwecke der Erfindung Gerinnungshemmer durch Chelatbildner gebildet sein. Zur Verwendung als Stabilisierungsmittel für Urin als biologische Probe kann die Zusammensetzung die Puffersubstanz und Urotropin, als trockene Mischung, z.B. in Pulverform, oder bevorzugt in wässriger Lösung, aufweisen, optional mit zumindest einem Gerinnungshemmer und/oder Chelatbildner, oder daraus bestehen. Generell bevorzugt ist eine biologische Probe eine Probe eines Gewebes oder einer Körperflüssigkeit eines Tiers oder eines Menschen.

Die zumindest eine Puffersubstanz kann unter Citratpuffer, Acetatpuffer, MES (2-N-Morpholinoethanolsulfonsäure), PIPES (Piperazin-N,N'-bis-2-ethanosulfonsäure), MOPS (3-N-Morpholinopropansulfonsäure), Phosphatpuffer und Mischungen von zumindest zweien dieser ausgewählt sein oder daraus bestehen. Der zumindest eine Gerinnungshemmer kann z.B. Hirudin sein, optional in Mischung mit einem Chelatbildner. Bevorzugt ist der Gerinnungshemmer ein Chelatbildner, der z.B. unter Citrat und EDTA und Mischungen dieser ausgewählt sein kann oder daraus bestehen kann.

Die Zusammensetzung kann ohne eine zugesetzte Verbindung als Fänger für freies oder in Wasser gelöstes Formaldehyd, insbesondere ohne Glycin, sein und/oder ohne zusätzlichen Chelatbildner, insbesondere ohne EDTA (Ethylendiamintetraacetat), z.B. wenn die Zusammensetzung Citrat enthält. Besonders bevorzugt besteht die Zusammensetzung aus einer Lösung von Citratpuffer und Urotropin in Wasser. Zur Verwendung als Stabilisierungsmittel für zellfreie Nukleinsäuren und/oder für Zellen in Urin bzw. in einem Verfahren zur Stabilisierung von zellfreien Nukleinsäuren und/oder von Zellen in Urin kann die Zusammensetzung aus Citratpuffer und Urotropin, optional mit einem Gerinnungshemmer und/oder Chelatbildner, in trockener Mischung bestehen, in der der Citratpuffer eingestellt ist, in der Probe auf einen pH-Wert von 7 oder darunter, bevorzugt 3,5 bis 7,0 zu puffern, insbesondere für ein vorbestimmtes Probenvolumen.

Die Puffersubstanz weist bevorzugt eine Pufferkapazität einer Citratpuffer-Lösung auf, die eine Konzentration im Bereich von 0,3 bis 1 M, bevorzugter 0,4 bis 0,7 M, zum Beispiel 0,5 M aufweist, und einen pH-Wert im Bereich von 3,5 bis 7, bevorzugt 4 bis 6,5, für Vollblut als Probe z.B. pH 4 bis 4,5, generell bevorzugter einen pH-Wert von 4,5 oder 4,2, und ist z.B. eingerichtet, die Mischung aus der biologischen Probe und der Zusammensetzung auf diesen pH-Wert zu puffern. Der Gehalt an Urotropin (Hexamethylentetramin) ist bevorzugt im Bereich von 1 bis 30 Gew./Vol.-%, bevorzugt 2 oder 5 bis 25 Gew./Vol.-% oder bis 20 Gew./Vol.-%, z.B. 5 bis 19 oder bis 8 Gew./Vol.-% in der Puffer-Lösung. Bevorzugt ist die Puffersubstanz Citratpuffer einer Konzentration im Bereich von 0,3 bis 1 M, bevorzugter 0,4 bis 0,7 M, zum Beispiel 0,5 M, und weist einen pH-Wert im Bereich von 3,5 bis 7, bevorzugt pH 4 bis 6,0, für Vollblut als Probe z.B. pH 4 bis 4,5, generell bevorzugter einen pH-Wert von 4,5 oder 4,2 auf, und ist bevorzugt eingerichtet, die Mischung aus der biologischen Probe und der Zusammensetzung auf diesen pH-Wert zu puffern.

Es hat sich gezeigt, dass der Citratpuffer die Koagulation von Vollblut ausreichend hemmt, auch ohne EDTA zu enthalten. Daher kann die zumindest eine Puffersubstanz und der zumindest eine Chelatbildner aus Citratpuffer gebildet sein.

Bevorzugt, insbesondere für Vollblut als biologische Probe, wird die erfindungsgemäße Zusammensetzung durch Herstellen des Puffers, der bevorzugt Citratpuffer ist, in Wasser, Einstellen des pH-Werts und anschließendes Zugeben und Lösen des Urotropins erzeugt. Bevorzugt wird der Puffer durch Mischen einer Lösung der Puffersubstanz, die z.B. Citronensäure oder Essigsäure ist, in Wasser mit einer Lösung eines Salzes der Puffersubstanz, z.B. Trinatrium-Citrat oder Natrium-Acetat, in Wasser in jeweils der gewünschten Konzentration des Puffers hergestellt, z.B. Citratpuffer bzw. Acetatpuffer.

Alternativ werden die Säure und das Salz der Säure in einem Verhältnis trocken vermischt, so dass sich bei Flüssigkeitszugabe der gewünschte pH-Wert einstellt.

Die erfindungsgemäße Verwendung der Zusammensetzung als Stabilisierungsmittel ist für biologische Proben, insbesondere Vollblut oder Urin, insbesondere für in einer biologischen Probe enthaltene zellfreie Nukleinsäure, DNA und/oder RNA, geeignet, mit der anschließenden Isolierung einer zellfreien Fraktion, z.B. von zellfreiem Plasma, bzw. von Zellen. Aus der zellfreien Fraktion können insbesondere zellfreie Nukleinsäuren analysiert werden. Dabei hat sich gezeigt, dass die Zusammensetzung geeignet ist, in biologischen Proben, insbesondere Vollblut, die Menge und die Struktur zellfreier Nukleinsäuren, insbesondere von zellfreier DNA oder zellfreier RNA zu stabilisieren und im Wesentlichen Veränderungen der Menge oder Struktur dieser Nukleinsäuren zu verhüten, so dass durch die Zusammensetzung im Wesentlichen keine Veränderungen der Menge oder Struktur dieser Nukleinsäuren verursacht werden, die spätere Analyse der Nukleinsäuren stören würden. Eine spätere Analyse der Nukleinsäuren kann beispielsweise durch Hybridisieren, Sequenzieren oder Amplifizieren, z.B. PCR erfolgen.

Der optionale Gehalt an PEG, z.B. eines oder eine Mischung von PEG 6000 bis PEG 20000, wirkt einer Hämolyse entgegen. Die Zusammensetzung enthält insbesondere bei der Verwendung zur späteren Analyse von zellfreier Nukleinsäure, bevorzugt mit Isolieren von Nukleinsäuren durch Adsorption an ein Adsorptionsmittel für Nukleinsäuren, kein PEG.

Die Zellen einer biologischen Probe, die mit der erfindungsgemäßen Zusammensetzung gemischt wurde, zeichnen sich dadurch aus, dass deren Gehalt an Nukleinsäuren, insbesondere DNA und RNA, im Wesentlichen unverändert bleibt und nicht den Gehalt an Nukleinsäuren der zellfreien Fraktion beeinflusst.

Die erfindungsgemäße Zusammensetzung hat den Vorteil, dass sie lagerstabil ist, beispielsweise für zumindest einen Monat, bevorzugter zumindest zwei Monate, z.B. für bis zu acht Monate oder bis zu sechs Monate bei 0 bis 30 °C, bevorzugter bei 5 bis 20 °C, um die stabilisierende Wirkung zu erzielen. Überdies ist die Zusammensetzung auch lagerstabil, wenn sie in einer Blutentnahmeröhre enthalten ist und darin für das einzuziehende Blut vorgelegt ist, bzw. in einem Urinprobengefäß enthalten ist und darin für einzufüllenden Urin vorgelegt ist. Entsprechend betrifft die Erfindung auch die Verwendung einer Blutentnahmeröhre zur Stabilisierung zellfreier Nukleinsäuren und/oder der Zellen einer biologischen Probe, die eine Vollblutprobe ist, wobei die Zusammensetzung in der Blutentnahmeröhre enthalten ist. Entsprechend betrifft die Erfindung auch die Verwendung eines Urinprobengefäßes zur Stabilisierung zellfreier Nukleinsäuren und/oder der Zellen der biologischen Probe, die eine Urinprobe ist, wobei die Zusammensetzung in dem Urinprobengefäß enthalten ist.

Es hat sich gezeigt, dass die Zusammensetzung ohne einen Gehalt an einer Fängerverbindung für freies oder in Wasser gelöstes Formaldehyd und ohne einen zusätzlichen Gehalt an einem Antikoagulans, wie beispielsweise EDTA, zur Stabilisierung von Nukleinsäuren und/oder Zellen geeignet ist. Gegenwärtig wird die Lagerstabilität der Zusammensetzung und ihre stabilisierende Wirkung auf biologische Proben darauf zurückgeführt, dass das Urotropin in der Zusammensetzung und in der Mischung mit der Probe im Gleichgewicht mit freiem Formaldehyd vorliegt, dessen Konzentration für die Stabilisierung bzw. Inaktivierung von Proteinen ausreicht.

Bevorzugt ist die Zusammensetzung in einer Blutentnahmeröhre enthalten, z.B. in einem Volumenanteil von maximal 20%, zumindest 2%, bevorzugt 5 bis 15%, insbesondere 8 bis 12%, z.B. zu 10% des Volumens an Vollblut, das in die Blutentnahmeröhre eingezogen werden kann, bzw. für das die Blutentnahmeröhre maximal eingerichtet ist. Bevorzugt ist die Zusammensetzung in einem Urinprobengefäß enthalten, z.B. in einem Volumenanteil von maximal 20%, zumindest 2%, bevorzugt 5 bis 15%, insbesondere 8 bis 12%, z.B. zu 10% des Volumens an Urin, das in das Urinprobengefäß eingefüllt werden kann, bzw. für das das Urinprobengefäß maximal eingerichtet ist.

Das Verfahren zur Stabilisierung einer biologischen Probe, die insbesondere Vollblut ist, weist die Schritte des
- Kontaktierens der Probe mit der Zusammensetzung zur Herstellung einer Mischung aus der Probe und der Zusammensetzung auf, bevorzugt in einem Volumenverhältnis von maximal 20%, bevorzugt 5 bis 15%, insbesondere 8 bis 12% der Zusammensetzung, die bevorzugt in wässriger Lösung ist, zur Probe,
- optionalen Lagerns der Mischung aus Probe und Zusammensetzung oberhalb von 0 °C, z.B. bei 0 bis 37 °C, z.B. bei 0 bis 30 °C, bevorzugter bei 5 bis 20 °C oder bei 22,5 °C, z.B. für 1h bis 14d, bevorzugt 5h bis 5d oder bis 3d oder bis 2d,
- optional mit anschließendem Auftrennen der Mischung in eine zellhaltige Fraktion und eine zellfreie Fraktion,
- bevorzugt des Zugebens von Proteinase, z.B. Proteinase K, nach dem Auftrennen der Mischung zu der erzeugten zellfreien Fraktion, und
- des Analysierens der Nukleinsäuren der Mischung aus deren zellfreien Fraktion und/oder des Analysierens der Zellen aus der zellhaltigen Fraktion.

Das Verfahren zur Stabilisierung und Analyse zellfreier Nukleinsäuren einer biologischen Probe weist z.B. die Schritte des Kontaktierens der Probe mit der Zusammensetzung zur Verwendung als Stabilisierungsmittel für die in der Probe enthaltenen zellfreien Nukleinsäure zur Herstellung einer Mischung aus der Probe und der Zusammensetzung und des Lagerns der Mischung aus der Probe und der Zusammensetzung bei 0 bis 30 °C für zumindest 1h bis 14d auf, bevorzugt mit anschließendem Auftrennen der Mischung in eine zellhaltige Fraktion und eine zellfreie Fraktion, bevorzugt des Zugebens von Proteinase, z.B. Proteinase K, nach dem Auftrennen der Mischung zu der erzeugten zellfreien Fraktion, und des Analysierens der Nukleinsäuren der Mischung aus deren zellfreien Fraktion und/oder des Analysierens der Zellen aus der zellhaltigen Fraktion, oder besteht aus diesen Schritten.

Das Analysieren der Mischung umfasst optional das Isolieren von Nukleinsäuren, bevorzugt durch Kontaktieren mit einem Adsorptionsmittel für Nukleinsäuren und anschließendem Waschen des Adsorptionsmittels und Eluieren gebundener Nukleinsäuren von dem Adsorptionsmittel. Entsprechende Adsorptionsmittel, z.B. Ionentauschermaterialien, sind z. B. in DNA-Isolierungskits von Macherey-Nagel (NucleoSnap DNA Plasma) oder Qiagen (QIAamp Circulating Nucleic Acid Kit) enthalten.

Das Auftrennen der Mischung in eine zellhaltige und eine zellfreie Fraktion kann generell z.B. durch Filtration oder Zentrifugation und/oder durch Adsorption erfolgen.

Generell, insbesondere für Urin als Probe, kann das Verfahren, insbesondere für die zellfreie Fraktion, zumindest einen Anreichungsschritt für freie Nukleinsäuren umfassen, z.B. das Ausfällen von Nukleinsäuren und/oder das Adsorbieren von Nukleinsäuren an ein Adsorbenz, z.B. an paramagnetische Partikel, die mit einem Adsorptionsmittel beschichtet sind.

Das Analysieren der zellhaltigen Fraktion kann optional das Anreichern von Zellen aus der zellhaltigen Fraktion umfassen, z.B. das Adsorbieren von Zellen an ein Adsorbenz, z.B. an paramagnetische Partikel, die mit einem Adsorptionsmittel, z.B. einem Antikörper, beschichtet sind.

Optional kann eine zellhaltige Fraktion, von der die zellfreie Fraktion aus einer Mischung der biologischen Probe mit der Zusammensetzung abgetrennt ist, in einer wässrigen Zusammensetzung, wie sie vorliegend als Stabilisierungsmittel beschrieben ist, suspendiert und aufgeschlossen werden.

Die Erfindung wird nun genauer anhand eines Beispiels mit Bezug auf die Figuren beschrieben, die in
- Figur 1 das Ergebnis einer PCR-Amplifikation zellfreier DNA aus einer Probe nach verschiedenen Lagerdauern und in
- Figur 2 das Ergebnis von PCR-Amplifikationen zweier DNA-Abschnitte als Quotient der Konzentrationen der Amplifikate nach verschiedenen Lagerdauern zeigen.

### Beispiel 1: Isolieren und Analysieren zellfreier DNA aus Vollblut

Die Zusammensetzung wurde durch Mischen von 0,5 M Citronensäure (Monohydrat) in Wasser und 0,5 M Trinatrium-Citrat (Dihydrat) in Wasser bis zum Erreichen von pH 4,2 und anschließendem Zugeben und Lösen von 18 Gew./Vol.-% Urotropin hergestellt.

Als Beispiel für eine biologische Probe wurde Vollblut zu 4,9 mL von 3 Spendern in jeweils 4 identische Blutentnahmeröhren eingezogen, die 0,49 mL Zusammensetzung (10 Vol.-% des Vollbluts) enthielten. Von diesen Blutentnahmeröhren, die diese Mischung aus Vollblut und der Zusammensetzung enthielten und die bei 22,5 °C gelagert wurden, wurde jeweils eine an Tag 0 (T0), Tag 3 (T3), Tag 7 (T7) und Tag 14 (T14) aufgearbeitet und daraus zellfreie DNA isoliert und analysiert. Dazu wurde aus jeder Blutentnahmeröhre eine zellfreie Fraktion durch einen zweistufigen Zentrifugationsprozess (10 min, 2.000 x g; 15 min, 15.000 x g) erzeugt.

Aus dem so erzeugten zellfreien Plasma, das die Zusammensetzung zur Stabilisierung enthielt, wurde mit dem NucleoSnap DNA Plasma Kit (erhältlich von Macherey-Nagel) zellfreie DNA isoliert. Im Unterschied zur Anweisung des Kits wurden 1,7 mL des zellfreien Plasmas statt 3,0 mL eingesetzt und der Lysepuffer VL sowie Ethanol ebenfalls von 3 mL auf 1,7 mL reduziert. Überdies wurde die Inkubationszeit für das zellfreie Plasma mit Proteinase K bei Raumtemperatur und bei 56 °C jeweils von 5 min auf 15 min verlängert.

Die Analyse der zellfreien DNA erfolgte durch Amplifikation des 115 bp Abschnitts ALU115 und des 247 bp Abschnitts ALU247 mittels quantitativer PCR wie von Umetani et al. (2006) beschrieben.

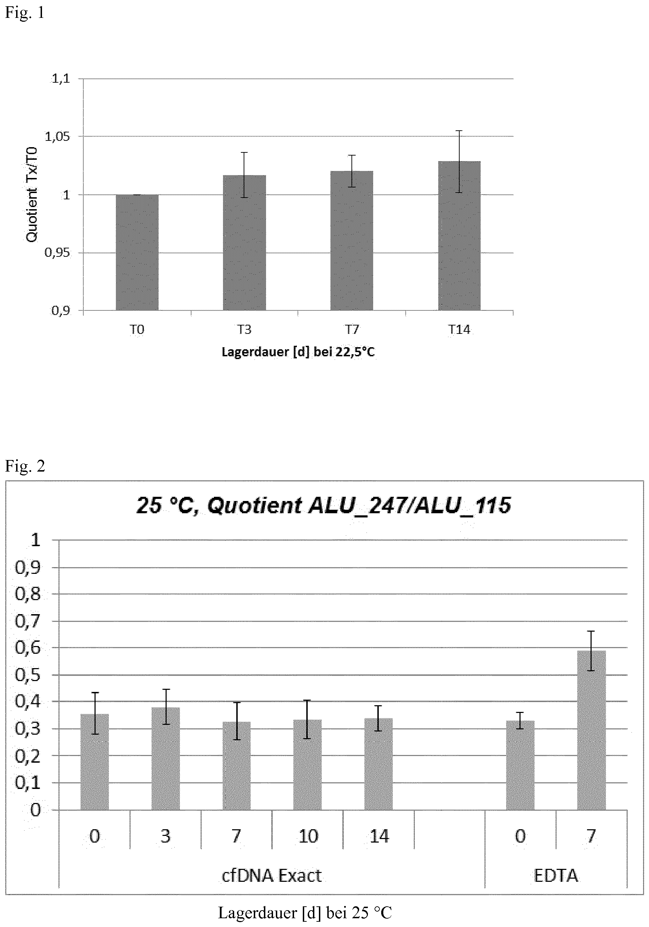

Die Figur 1 zeigt den Mittelwert der Quotienten aus der Konzentration von ALU115 zum angegebenen Zeitpunkt an Tag 0 (T0), Tag 3 (T3 = Tx), Tag 7 (T7 = Tx) und Tag 14 (T14 = Tx) der Aliquots und der Konzentration von ALU115 an Tag 0 (T0). Dabei zeigt ein Quotient (Tx/T0) von 1 an, dass die Konzentration der zellfreien DNA in der Mischung unverändert bzw. stabil bleibt und auch, dass die zellfreie DNA der Mischung unverändert durch PCR amplifizierbar ist. Dieses Ergebnis zeigt, dass die zellfreie DNA in der Mischung aus Vollblut und der Zusammensetzung über eine Lagerdauer von zumindest 14 d bei 22,5 °C in Konzentration und Struktur für die anschließende Analyse stabilisiert wird.

Die Figur 2 zeigt die Quotienten der absoluten Mengen zellfreier DNA (cfDNA) am Beispiel von ALU 247 und ALU 115 an den angegebenen Zeitpunkten der Lagerung bei 25 °C (Tag 0 (0), Tag 3 (3), Tag 7 (7), Tag 10 (10) und Tag 14 (14) für die erfindungsgemäße Zusammensetzung (cfDNA Exact) und für EDTA-Proben (EDTA) an Tag 0 (0) und Tag 7 (7)). Das Ergebnis zeigt die Stabilisierung anhand des über die Inkubationszeit konstanten Quotienten durch die erfindungsgemäße Zusammensetzung (Exact). In nicht stabilisierten, EDTA-antikoagulierten Proben steigt der ALU 247 / ALU 115-Quotient mit zunehmender Lagerdauer an. Eine ungenügende Stabilisierung von Blutproben / biologischen Proben führt zur Lyse von Zellen und damit zur Freisetzung der genomischen DNA (gDNA). Ein ALU 247 / ALU 115 - Quotient von 1, also gleiche Mengen ALU 247 und ALU 115, ist charakteristisch für gDNA.

Diese Ergebnisse zeigen auch, dass die Zusammensetzung für die spätere Isolierung von zellfreien Nukleinsäuren aus einer zellfreien Mischung der Zusammensetzung mit Plasma mit der Adsorption der Nukleinsäuren an ein Adsorptionsmittel geeignet ist, bzw. diese Isolierung nicht beeinträchtigt.

### Beispiel 2: Isolieren und Analysieren von Zellen aus Vollblut

Entsprechend Bsp. 1 wurde eine Mischung aus der Zusammensetzung und Vollblut hergestellt und bei 22,5°C gelagert. Eine zweite Vollblutprobe enthielt anstelle der erfindungsgemäßen Zusammensetzung physiologische Kochsalzlösung und EDTA (1,6 mg / mL Blut) zur Verhinderung der Koagulation. Von beiden Arten von Vollblutproben wurden an Tag 0, Tag 1, Tag 3 und Tag 5 je 5,0 mL einer Erythrocyten-Lyse unterzogen und die zirkulierenden Endothelzellen (cEC) mit Hilfe des cEC Enrichment and Enumeration Kit der Firma Miltenyi Biotech angereichert und analysiert. Bei gesunden Menschen liegt die Zahl der zirkulierenden Endothelzellen bei ca. 1 - 20 Zellen / mL. Bei unterschiedlichen Erkrankungen ist die Zahl der cECs teilweise erheblich erhöht. Stabilisierende Präparate müssen gewährleisten, dass die Anzahl von cEC in gelagerten Blutproben stabil ist. Nach der für das Kit beschriebenen Analyse zeigte sich, dass die Anzahl der nachgewiesenen CEC in nur den erfindungsgemäß stabilisierten Proben über die Lagerzeit konstant blieb.

### Beispiel 3: Isolieren und Analysieren zellfreier DNA aus Urin

Die Zusammensetzung wurde durch Mischen von 0,5 M Citronensäure (Monohydrat) in Wasser und 0,5 M Trinatrium-Citrat (Dihydrat) in Wasser bis zum Erreichen von pH 4,2 und anschließendem Zugeben und Lösen von 15 Gew./Vol.-% Urotropin hergestellt.

Als Beispiel für eine biologische Probe wurden 90 mL Urin unmittelbar nach der Probenahme mit 1/10 Vol. der Zusammensetzung gemischt. Aus dieser Mischung, die bei 22,5 °C gelagert wurde, wurde jeweils ein Aliquot an Tag 0, Tag 3, Tag 7 und Tag 14 aufgearbeitet und daraus zellfreie DNA isoliert und analysiert. Dazu wurde aus einem Aliquot jeweils eine zellfreie Fraktion durch eine Zentrifugation bei 15000 x g für 15 min und Abtrennen der zellfreien Fraktion erzeugt. Aus der so erzeugten zellfreien Fraktion, die die Zusammensetzung zur Stabilisierung enthielt, wurde mit dem NucleoSnap DNA Plasma Kit (erhältlich von Macherey-Nagel) zellfreie DNA isoliert.

Es hat sich im Vergleich zu Urinproben, die unbehandelt oder mit Zusatz eines gleichen Volumens physiologischer Kochsalzlösung oder nur der gelösten Puffersubstanz versetzt und identisch gelagert wurden, gezeigt, dass die zellfreie DNA durch die Zusammensetzung stabilisiert wurde.

### Beispiel 4: Analyse von stabilisierten Zellen im Urin

Entsprechend Bsp. 3 wurde Urin mit Prostata-Karzinomzellen (LnCap) versetzt und zur Stabilisierung mit der Zusammensetzung bzw. zum Vergleich mit physiologischer Kochsalzlösung vermischt und gelagert.

Die Analyse erfolgte nach identischer Lagerzeit der Vergleichsproben an durch Zentrifugation sedimentierten Zellen. Es zeigte sich, dass nur die erfindungsgemäße Zusammensetzung den durchflusszytometrischen Nachweis der zugesetzten Zellen erlaubte.

Es hat sich im Vergleich zu Urinproben, die unbehandelt oder mit Zusatz eines gleichen Volumens physiologischer Kochsalzlösung oder nur der gelösten Puffersubstanz versetzt und identisch gelagert wurden, gezeigt, dass die Zusammensetzung Oberflächenmarker der Zellen während der Lagerung stabilisierte.

## Patentansprüche

1. Verwendung einer Zusammensetzung als Stabilisierungsmittel für die in einer biologischen Probe enthaltenen zellfreien Nukleinsäuren, **dadurch gekennzeichnet, dass** die Zusammensetzung zumindest eine Puffersubstanz, die auf einen pH-Wert von 7 oder darunter puffert, zumindest einen Gerinnungshemmer, der bevorzugt ein Chelatbildner ist, und Urotropin, optional PEG, in wässriger Lösung aufweist.

2. Verwendung nach Anspruch 1, **dadurch gekennzeichnet, dass** die Zusammensetzung aus der zumindest einen Puffersubstanz, dem zumindest einen Gerinnungshemmer, der ein Chelatbildner ist, und Urotropin, optional PEG, in wässriger Lösung besteht.

3. Verwendung einer Zusammensetzung als Stabilisierungsmittel für die in einer biologischen Probe enthaltenen zellfreien Nukleinsäuren, **dadurch gekennzeichnet, dass** die Zusammensetzung zumindest eine Puffersubstanz, die eingerichtet ist, die biologische Probe auf einen pH-Wert von 7 oder darunter zu puffern, zumindest einen Gerinnungshemmer, der bevorzugt ein Chelatbildner ist, und Urotropin, optional PEG, als trockene Mischung aufweist.

4. Verwendung nach einem der voranstehenden Ansprüche, **dadurch gekennzeichnet, dass** die Zusammensetzung keine Fängerverbindung für freies oder gelöstes Formaldehyd enthält.

5. Verwendung nach einem der voranstehenden Ansprüche, **dadurch gekennzeichnet, dass** die zumindest eine Puffersubstanz die wässrige Lösung auf einen pH-Wert im Bereich von 3,5 bis 7 puffert und eine Pufferkapazität aufweist, die einer Konzentration von 0,3 bis 1 M Citrat entspricht, und in der Lösung 1 bis 30 Gew./Vol.-% Urotropin gelöst ist.

6. Verwendung nach einem der voranstehenden Ansprüche, **dadurch gekennzeichnet, dass** die zumindest eine Puffersubstanz und der zumindest eine Gerinnungshemmer, der ein Chelatbildner ist, durch Citratpuffer gebildet sind.

7. Verwendung nach einem der voranstehenden Ansprüche, **dadurch gekennzeichnet, dass** die zumindest eine Puffersubstanz unter Citratpuffer, Acetatpuffer, MES (2-N-Morpholinoethanolsulfonsäure), PIPES (Piperazin-N,N'-bis-2-ethanosulfonsäure), MOPS (3-N-Morpholinopropansulfonsäure), Phosphatpuffer und Mischungen von zumindest zweien dieser ausgewählt ist und der zumindest eine Gerinnungshemmer ein Chelatbildner ist, der unter Citrat und EDTA und Mischungen dieser ausgewählt ist.

8. Verwendung nach einem der voranstehenden Ansprüche, **gekennzeichnet durch** die Verwendung als Stabilisierungsmittel für zellfreie Nukleinsäuren, das anschließende Isolieren einer zellfreien Fraktion und die Analyse der in der zellfreien Fraktion enthaltenen Nukleinsäuren umfasst.

9. Verwendung nach einem der voranstehenden Ansprüche, **gekennzeichnet durch** die zusätzliche Verwendung als Stabilisierungsmittel für in der biologischen Probe enthaltene Zellen, und das anschließende Isolieren einer zellhaltigen Fraktion und die Analyse der darin enthaltenen Zellen umfasst.

10. Verwendung nach Anspruch 9, **dadurch gekennzeichnet, dass** die Analyse der Zellen der zellhaltigen Fraktion die Analyse der in Zellen enthaltenen Nukleinsäuren und/oder die immunologische Analyse von zelloberflächengebundenen Proteinen umfasst.

11. Verwendung nach einem der voranstehenden Ansprüche, **gekennzeichnet dadurch, dass** die biologische Probe Vollblut ist und die Zusammensetzung in einem volumetrischen Anteil von bis zu 20% an dem maximal einzuziehenden Probenvolumen, für die eine Blutentnahmeröhre eingerichtet ist, in der Blutentnahmeröhre enthalten ist.

12. Verwendung nach einem der Ansprüche 1 bis 10, **gekennzeichnet dadurch, dass** die biologische Probe Urin ist.

13. Verwendung nach einem der voranstehenden Ansprüche, **gekennzeichnet dadurch, dass** die biologische Probe in Mischung mit der Zusammensetzung bei 0 bis 37 °C für 1h bis 14d stabil ist.

14. Verwendung nach einem der voranstehenden Ansprüche , **dadurch gekennzeichnet, dass** die Zusammensetzung aus Citratpuffer, der auf einen pH-Wert im Bereich von 3,5 bis 7 puffert, zumindest einen Gerinnungshemmer, der ein Chelatbildner für Calciumionen ist, und Urotropin, optional PEG, in wässriger Lösung besteht.

15. Verwendung nach Anspruch 14, **dadurch gekennzeichnet, dass** die Zusammensetzung aus Citratpuffer als Puffersubstanz und als Chelatbildner und Urotropin in wässriger Lösung besteht und der Citratpuffer auf einen pH-Wert im Bereich von 3,5 bis 7 puffert und in der Lösung 1 bis 30 Gew./Vol.-% Urotropin gelöst ist.

16. Verwendung einer Blutentnahmeröhre zur Stabilisierung zellfreier Nukleinsäuren einer biologischen Probe, **dadurch gekennzeichnet, dass** sie eine Zusammensetzung zur Verwendung als Stabilisierungsmittel für die in einer biologischen Probe enthaltenen zellfreien Nukleinsäuren nach einem der voranstehenden Ansprüche in einem volumetrischen Anteil von bis zu 20% an dem maximal in die Blutentnahmeröhre einzuziehenden Probenvolumen enthält.

17. Verwendung eines Urinprobengefäßes zur Stabilisierung zellfreier Nukleinsäuren einer Urinprobe, **dadurch gekennzeichnet, dass** sie eine Zusammensetzung zur Verwendung als Stabilisierungsmittel für die in einer biologischen Probe enthaltenen zellfreien Nukleinsäuren nach einem der Ansprüche 1 bis 15 enthält.

18. Verfahren zur Stabilisierung und Analyse zellfreier Nukleinsäuren einer biologischen Probe mit den Schritten des
- Kontaktierens der Probe mit einer Zusammensetzung zur Verwendung nach einem der Ansprüche 1 bis 14 zur Herstellung einer Mischung aus der Probe und der Zusammensetzung und
- des Lagerns der Mischung aus der Probe und der Zusammensetzung bei 0 bis 37 °C für 1h bis 14d.

19. Verfahren nach Anspruch 18, **dadurch gekennzeichnet, dass** die Zusammensetzung in einem Volumenverhältnis von maximal 20% des Volumens der Probe mit der Probe kontaktiert wird.

20. Verfahren nach einem der Ansprühe 18 bis 19, **gekennzeichnet durch** die Schritte
- des an das Lagern anschließenden Auftrennens der Mischung in eine zellhaltige Fraktion und eine zellfreie Fraktion, und
- des Analysierens der Nukleinsäuren der zellfreien Fraktion.

21. Verfahren nach Anspruch 20, **gekennzeichnet durch** den Schritt des Analysierens der Nukleinsäuren in der zellhaltigen Fraktion.

22. Verfahren nach einem der Ansprüche 20 bis 21, **gekennzeichnet durch** den Schritt des Analysierens von zelloberflächengebundenen Proteinen in der zellhaltigen Fraktion.

23. Verfahren nach einem der Ansprüche 20 bis 21, **dadurch gekennzeichnet, dass** nach dem Auftrennen der Mischung zu der zellfreien Fraktion Proteinase zugegeben wird und die Mischung anschließend inkubiert wird.

24. Verfahren nach einem der Ansprüche 20 bis 21, **dadurch gekennzeichnet, dass** aus der zellfreien Fraktion Nukleinsäuren isoliert und anschließend analysiert werden.

25. Verfahren nach einem der Ansprüche 20 bis 22, **dadurch gekennzeichnet, dass** die biologische Probe Vollblut oder Urin ist.

## Claims

1. Use of a composition as a stabilising agent for the cell-free nucleic acids contained in a biological sample, **characterized in that** the composition in aqueous solution comprises at least one buffering compound that buffers to a pH value of 7 or below, at least one anticoagulant that preferably is a chelating agent, and urotropin, optionally PEG.

2. Use according to claim 1, **characterized in that** the composition consists of the at least one buffering compound, the at least one anticoagulant that is a chelating agent, and urotropin, optionally PEG, in aqueous solution.

3. Use of a composition as a stabilising agent for the cell-free nucleic acids contained within a biological sample, **characterized in that** the composition comprises, as a dry mixture, at least one buffering compound that is configured to buffer the biological sample to a pH value of 7 or below, at least one anticoagulant that preferably is a chelating agent, and urotropin, optionally PEG.

4. Use according to one of the preceding claims, **characterized in that** the composition contains no quenching compound for free or dissolved formaldehyde.

5. Use according to one of the preceding claims, **characterized in that** the at least one buffering compound buffers the aqueous solution to a pH value in the range of 3.5 to 7 and has a buffer capacity that is equal to a concentration of 0.3 to 1 M citrate, and **in that** 1 to 30 wt./vol.-% urotropin is dissolved in the solution.

6. Use according to one of the preceding claims, **characterized in that** the at least one buffering substance and the at least one anticoagulant that is a chelating agent are formed by citrate buffer.

7. Use according to one of the preceding claims, **characterized in that** the at least one buffering compound is selected from citrate buffer, acetate buffer, MES (2-N-morpholinoethanolsulfonic acid), PIPES (piperazine-N,N'-bis-2-ethanolsulfonic acid), MOPS (3-N-morpholinopropanesulfonic acid), phosphate buffer, and mixtures of at least two of these, and **in that** the at least one anticoagulant is a chelating agent selected from citrate, and EDTA, and mixtures thereof.

8. Use according to one of the preceding claims, **characterized by** the use as a stabilizing agent for cell-free nucleic acids that comprises the subsequent isolation of a cell-free fraction and the analysis of the nucleic acids contained in the cell-free fraction.

9. Use according to one of the preceding claims, **characterized by** the additional use as a stabilising agent for cells contained in the biological sample, and comprising the subsequent isolation of a cell-containing fraction and the analysis of the cells contained therein.

10. Use according to claim 9, **characterized in that** the analysis of the cells of the cell-containing fraction comprises the analysis of the nucleic acids contained within the cells and/or the immunological analysis of cell surface-bound proteins.

11. Use according to one of the preceding claims, **characterized in that** the biological sample is whole blood and **in that** the composition is contained in a blood collection tube in a volume fraction of up to 20 % of the maximum sample volume to be drawn, for which the blood collection tube is configured.

12. Use according to one of claims 1 to 10, **characterized in that** the biological sample is urine.

13. Use according to one of the preceding claims, **characterized in that** the biological sample in a mixture with the composition is stable at 0 to 37 °C for 1 h to 14 d.

14. Use according to one of the preceding claims, **characterized in that** the composition consists of citrate buffer that buffers to a pH value in the range of 3.5 to 7, at least one anticoagulant that is a chelating agent for calcium ions, and urotropin, optionally PEG, in aqueous solution.

15. Use according to claim 14, **characterized in that** the composition consists of citrate buffer as buffering compound and as chelating agent and of urotropin in aqueous solution, and **in that** the citrate buffer buffers to a pH value in the range of 3.5 to 7, and **in that** in the solution 1 to 30 wt/vol.-% urotropin is dissolved.

16. Use of a blood collection tube for stabilising cell-free nucleic acids of a biological sample, **characterized in that** it contains a composition for use as stabilising agent for the cell-free nucleic acids contained in a biological sample according to one of the preceding claims in a volume fraction of up to 20 % of the maximum sample volume to be drawn into the blood collection tube.

17. Use of a urine sample container for stabilising cell-free nucleic acids of a urine sample, **characterized in that** it contains a composition for use as stabilising agent for the cell-free nucleic acids contained in a biological sample according to one of claims 1 to 15.

18. Process for stabilisation and analysis of cell-free nucleic acids of a biological sample having the steps of
- contacting the sample with a composition for use according to one of claims 1 to 14 for producing a mixture of the sample and the composition and
- storing the mixture of the sample and the composition at 0 to 37°C for 1 h to 14 d.

19. Process according to claim 18, **characterized in that** the composition is contacted with the sample in a volume fraction of at maximum 20 % of the sample volume.

20. Process according to one of claims 18 to 19, **characterized by** the steps of
- subsequent to the storing, separating the mixture into a cell-containing fraction and a cell-free fraction and
- analysing the nucleic acids of the cell-free fraction.

21. Process according to claim 20, **characterized by** the step of analysing the nucleic acids in the cell-containing fraction.

22. Process according to one of claims 20 to 21, **characterized by** the step of analysing cell surface-bound proteins in the cell-containing fraction.

23. Process according to one of claims 20 to 21, **characterized in that** subsequent to separating the mixture into the cell-free fraction, proteinase is added and the mixture is subsequently incubated.

24. Process according to one of claims 20 to 21, **characterized in that** nucleic acids are isolated from the cell-free fraction and are subsequently analysed.

25. Process according to one of claims 20 to 22, **characterized in that** the biological sample is whole blood or urine.

## Revendications

1. Utilisation d'une composition comme agent stabilisateur pour les acides nucléiques acellulaires contenus dans un échantillon biologique, **caractérisée en ce que** la composition comprend au moins une substance tampon qui tamponne à un pH inférieur ou égal à 7, au moins un inhibiteur de coagulation, qui est de préférence un agent chélatant, et de l'urotropine, optionnel du PEG, en solution aqueuse.

2. Utilisation selon la revendication 1, **caractérisée en ce que** la composition est constituée de l'au moins une substance tampon, dudit au moins un inhibiteur de coagulation qui est un agent chélatant, et de l'urotropine, optionnel du PEG, en solution aqueuse.

3. Utilisation d'une composition comme agent stabilisant pour les acides nucléiques acellulaires contenus dans un échantillon biologique, **caractérisée en ce que** la composition comprend, sous forme de mélange sec, au moins une substance tampon adaptée pour tamponner l'échantillon biologique à un pH inférieur ou égal à 7, au moins un inhibiteur de coagulation, qui est de préférence un agent chélatant, et de l'urotropine, optionnel du PEG.

4. Utilisation selon l'une des revendications précédentes, **caractérisée en ce que** la composition ne contient pas de composé de capture du formaldéhyde libre ou dissous.

5. Utilisation selon l'une des revendications précédentes, **caractérisée en ce que** l'au moins une substance tampon tamponne la solution aqueuse à un pH compris entre 3,5 et 7 et présente une capacité tampon correspondant à une concentration de 0,3 à 1 M de citrate, et **en ce que** 1 à 30 % p/v d'urotropine est dissous dans la solution.

6. Utilisation selon l'une des revendications précédentes, **caractérisée en ce que** l'au moins une substance tampon et l'au moins un inhibiteur de coagulation, qui est un agent chélatant, sont formés par des tampons de citrate.

7. Utilisation selon l'une des revendications précédentes, **caractérisée en ce que** l'au moins une substance tampon est choisie parmi le tampon citrate, le tampon acétate, le MES (acide 2-N-morpholinoéthanol sulfonique), le PIPES (acide pipérazine-N,N'-bis-2-éthanosulfonique), MOPS (acide 3-N-morpholinopropanesulfonique), un tampon phosphate et des mélanges d'au moins deux d'entre eux, et l'au moins un anticoagulant est un agent chélatant choisi parmi le citrate et l'EDTA et leurs mélanges.

8. Utilisation selon l'une des revendications précédentes, **caractérisée en ce qu'**elle comprend l'utilisation en tant qu'agent de stabilisation d'acides nucléiques acellulaires, l'isolement ultérieur d'une fraction acellulaire et l'analyse des acides nucléiques contenus dans la fraction acellulaire.

9. Utilisation selon l'une des revendications précédentes, **caractérisée par** l'utilisation supplémentaire en tant qu'agent stabilisant pour les cellules contenues dans l'échantillon biologique, et comprenant l'isolement ultérieur d'une fraction contenant des cellules et l'analyse des cellules contenues dans cette fraction.

10. Utilisation selon la revendication 9, **caractérisée en ce que** l'analyse des cellules de la fraction contenant des cellules comprend l'analyse des acides nucléiques contenus dans les cellules et/ou l'analyse immunologique des protéines liées à la surface des cellules.

11. Utilisation selon l'une des revendications précédentes, **caractérisée en ce que** l'échantillon biologique est du sang total et **en ce que** la composition est contenue dans le tube de prélèvement sanguin dans une proportion volumétrique allant jusqu'à 20 % du volume maximal d'échantillon à prélever, pour lequel un tube de prélèvement sanguin est aménagé.

12. Utilisation selon l'une des revendications 1 à 10, **caractérisée en ce que** l'échantillon biologique est de l'urine.

13. Utilisation selon l'une des revendications précédentes, **caractérisée en ce que** l'échantillon biologique est stable en mélange avec la composition entre 0 et 37 °C pendant 1 h à 14 d.

14. Utilisation selon l'une des revendications précédentes, **caractérisée en ce que** la composition est constituée d'un tampon citrate tamponnant à un pH compris entre 3,5 et 7, d'au moins un anticoagulant qui est un chélateur de l'ion calcium, et d'urotropine, optionnel de PEG, en solution aqueuse.

15. Utilisation selon la revendication 14, **caractérisée en ce que** la composition est constituée d'un tampon citrate en tant que substance tampon et en tant qu'agent chélatant et d'urotropine en solution aqueuse, et **en ce que** le tampon citrate tamponne à un pH dans la plage de 3,5 à 7 et **en ce que** 1 à 30 % p/v d'urotropine est dissous dans la solution.

16. Utilisation d'un tube de prélèvement sanguin pour stabiliser les acides nucléiques acellulaires d'un échantillon biologique, **caractérisée en ce qu'**elle contient une composition destinée à être utilisée comme agent de stabilisation pour les acides nucléiques acellulaires contenus dans un échantillon biologique selon l'une des revendications précédentes, dans une proportion volumétrique allant jusqu'à 20 % du volume maximal d'échantillon à introduire dans le tube de prélèvement sanguin.

17. Utilisation d'un récipient de prélèvement d'urine pour stabiliser les acides nucléiques acellulaires d'un échantillon d'urine, **caractérisée en ce qu'**elle comprend une composition destinée à être utilisée comme agent de stabilisation des acides nucléiques acellulaires contenus dans un échantillon biologique selon l'une des revendications 1 à 15.

18. Procédé de stabilisation et d'analyse d'acides nucléiques acellulaires d'un échantillon biologique, comprenant les étapes consistant à
- la mise en contact de l'échantillon avec une composition destinée à être utilisée selon l'une quelconque des revendications 1 à 14 pour produire un mélange de l'échantillon et de la composition, et
- le stockage du mélange de l'échantillon et de la composition entre 0 et 37 °C pendant 1 h à 14 d.

19. Procédé selon la revendication 18, **caractérisé en ce que** la composition est mise en contact avec l'échantillon dans un rapport volumique d'au plus 20 % du volume de l'échantillon.

20. Procédé selon l'une quelconque des revendications 18 à 19, **caractérisé par** les étapes
- de la séparation, après le stockage, du mélange en une fraction contenant des cellules et une fraction sans cellules, et
- l'analyse des acides nucléiques de la fraction acellulaire.

21. Procédé selon la revendication 20, **caractérisé par** l'étape d'analyse des acides nucléiques dans la fraction contenant des cellules.

22. Procédé selon l'une des revendications 20 à 21, **caractérisé par** l'étape d'analyse des protéines liées à la surface des cellules dans la fraction contenant les cellules.

23. Procédé selon l'une des revendications 20 à 21, **caractérisé en ce que**, après séparation du mélange, on ajoute de la protéinase à la fraction acellulaire, puis on fait incuber le mélange.

24. Procédé selon l'une quelconque des revendications 20 à 21, **caractérisé en ce que** des acides nucléiques sont isolés de la fraction acellulaire et sont ensuite analysés.

25. Procédé selon l'une des revendications 20 à 22, **caractérisé en ce que** l'échantillon biologique est du sang total ou de l'urine.
